# EUROPEAN PATENT SPECIFICATION

(11) **EP 4 326 356 B1**
(45) Date of publication and mention of the grant of the patent: **08.04.2026**
(21) Application number: 22792106.1
(22) Date of filing: 22.04.2022
(51) Int. Cl.: A61L 27/28, A61F 2/20, A61L 27/14

(54) **RHAMNOLIPID COATING OF MEDICAL DEVICES**
RHAMNOLIPIDBESCHICHTUNG VON MEDIZINISCHEN VORRICHTUNGEN
REVÊTEMENT PAR RHAMNOLIPIDES DE DISPOSITIFS MÉDICAUX

(30) Priority: 23.04.2021 SE 2150521
(43) Date of publication of application: 28.02.2024
(73) Proprietor: Coloplast A/S, 3050 Humlebaek (DK)
(72) Inventor: ALLEGRONE, Gianna, 10133 TORINO (IT); CARMAGNOLA, Irene, 14015 SAN DAMIANO D'ASTI (IT); CERESA, Chiara, 28076 POGNO (IT); CHIONO, Valeria, 19011 MONTARETTO-BONASSOLA (IT); CIARDELLI, Gianluca, 56125 PISA (IT); FRACCHIA, Letizia, 15068 POZZOLO FORMIGARO (IT)
(86) International application number: PCT/SE2022/050393
(87) International publication number: WO 2022/225444

(56) References cited:
- US-B1- 7 166 128
- US-B1- 7 166 128
- RODRIGUES, L. ET AL.: "Influence of Biosurfactants from Probiotic Bacteria on Formation of Biofilms on Voice Prostheses", APPLIED AND ENVIRONMENTAL MICROBIOLOGY, vol. 70, no. 7, 2004, pages 4408 - 4410, XP093001199
- L.R. RODRIGUES ET AL: "Interference in adhesion of bacteria and yeasts isolated from explanted voice prostheses to silicone rubber by rhamnolipid biosurfactants", JOURNAL OF APPLIED MICROBIOLOGY, vol. 100, no. 3, 1 March 2006 (2006-03-01), pages 470 - 480, XP055027016, ISSN: 1364-5072, DOI: 10.1111/j.1365-2672.2005.02826.x
- CERESA CHIARA ET AL: "Medical-Grade Silicone Coated with Rhamnolipid R89 Is Effective against Staphylococcus spp. Biofilms", vol. 24, no. 21, 1 November 2019 (2019-11-01), DE, pages 3843, XP093001184, ISSN: 1433-1373, Retrieved from the Internet <URL:https://www.ncbi.nlm.nih.gov/pmc/journals/3416> DOI: 10.3390/molecules24213843
- SILVA, S. F.F. P.: "Antimicrobial Glycolipids: Evaluation of their Antimicrobial Activity, Antibiofilm Activity and Mechanisms of Action", MASTER'S DISSERTATIONS, 20 January 2021 (2021-01-20), XP002812820, Retrieved from the Internet <URL:https://run.unl.pt/handle/10362/111052>
- RUINI FRANCESCA ET AL: "P828 Surface functionalization strategies to inhibit biofilm formation on silicone materials for implantable devices", EUROPEAN CELLS AND MATERIALS, vol. 33, no. Suppl. 2, 1 January 2017 (2017-01-01), XP093001188
- RODRIGUES, L. ET AL.: "Influence of Biosurfactants from Probiotic Bacteria on Formation of Biofilms on Voice Prostheses", APPLIED AND ENVIRONMENTAL MICROBIOLOGY, vol. 70, no. 7, 2004, pages 4408 - 4410
- L.R. RODRIGUES, BANAT I.M., MEI H.C., TEIXEIRA J.A., OLIVEIRA R.: "Interference in adhesion of bacteria and yeasts isolated from explanted voice prostheses to silicone rubber by rhamnolipid biosurfactants", JOURNAL OF APPLIED MICROBIOLOGY, ACADEMIC PRESS, vol. 100, no. 3, 1 March 2006 (2006-03-01), pages 470 - 480, XP055027016, ISSN: 13645072, DOI: 10.1111/j.1365-2672.2005.02826.x
- CERESA CHIARA, TESSAROLO FRANCESCO, MANIGLIO DEVID, TAMBONE ERICA, CARMAGNOLA IRENE, FEDELI EMANUELE, CAOLA IOLE, NOLLO GIANDOMENI: "Medical-Grade Silicone Coated with Rhamnolipid R89 Is Effective against Staphylococcus spp. Biofilms", MOLECULES, SPRINGER VERLAG, BERLIN, DE, vol. 24, no. 21, 1 November 2019 (2019-11-01), DE , pages 3843, XP093001184, ISSN: 1433-1373, DOI: 10.3390/molecules24213843
- SILVA, S. F.F. P.: "Antimicrobial Glycolipids: Evaluation of their Antimicrobial Activity, Antibiofilm Activity and Mechanisms of Action", MASTER'S DISSERTATIONS, 20 January 2021 (2021-01-20), Retrieved from the Internet <URL:https://run.unl.pt/handle/10362/111052>
- RUINI FRANCESCA, CERESA CHIARA, ALLEGRONE GIANNA, MANIGLIO DEVID, PICCOLI FEDERICO, RINALDI MAURIZIO, NOLLO GIANDOMENICO, CAOLA IO: "P828 Surface functionalization strategies to inhibit biofilm formation on silicone materials for implantable devices", EUROPEAN CELLS AND MATERIALS, vol. 33, no. Suppl. 2, 1 January 2017 (2017-01-01), XP093001188

## Description

### Technical Field

The present disclosure relates to voice prosthesis having irregular and/or curved silicone surfaces coated with rhamnolipids as well as methods for coating a medical device having irregular and/or curved silicone surfaces with rhamnolipids grafting.

### Background Art

Bacterial colonisation of synthetic materials surfaces constitutes a problem in many fields, for example in the field of implantable medical devices. The use of implantable medical devices is increasing over time. For implantable devices silicone rubber is widely used due to its biocompatibility properties, high mechanical strength and elasticity and potentially long device life time.

One problem commonly associated with implanted medical devices is the formation of biofilms on the surfaces of the device. Biofilms are communities of microorganisms where cells stick to each other and often also to a surface. These communities are embedded within a slimy extracellular matrix consisting of polymeric substance. The formation of biofilm on implanted medical devices is a common source of complications often involving loss of function of the implanted device and shortening of the device lifetime, leading to earlier exchange of the device.

Example of silicone devices on which the formation of biofilm is a known problem are permanent implants like breast implants, nasal implants, gastric bands, joint implants, gluteal implants, pelvic meshes etc. In the case of permanent implants, the formation of biofilm may cause device failure or device-associated infections leading to hospitalization and need for surgery. In devices for shorter duration implantation e.g. voice prostheses, silicone tubes for the nasolacrimal duct and tympanic ear tubes, the formation of biofilm can cause impaired device performance and device failure leading to the need for earlier exchange of the device.

Several surface treatments for silicone medical devices have been suggested to improve the resistance against biofilm formation, including antibiotic, antifungal or antimicrobial surface treatments, e.g. silver oxide or metallic silver, caspofungin peptides, selenium etc. However, the use of silver ions or silver nanoparticles in polymer matrices or quaternary amine compounds may be of concern with regards to damage to human cells and tissue.

L.R. Rodrigues ET AL: "Interference in adhesion of bacteria and yeasts isolated from explanted voice prostheses to silicone rubber by rhamnolipid biosurfactants", Journal of Applied Microbiology, vol. 100, no. 3, 1 March 2006 pages 470-480, ISSN: 1364-5072, DOI: 10.1111/j.1365-2672.2005.02826.x discloses the ability of rhamnolipid biosurfactant to inhibit adhesion of micro-organisms to silicone rubber. It was concluded that biosurfactant could be used to form an adsorbed rhamnolipid layer which prevents the microbial colonization of silicone rubber voice prosthesis. An object of the present disclosure is to provide a voice prosthesis having a coating preventing or reducing the formation of biofilms on the surface of the voice prosthesis as well as methods for coating a voice prosthesis with such a coating.

### Summary

Accordingly, the present invention preferably seeks to mitigate, alleviate or eliminate one or more of the above-identified deficiencies in the art and disadvantages singly or in any combination and solves at least the above mentioned problems by providing a voice prosthesis coated with rhamnolipids and by providing a method of coating a medical device with rhamnolipids.

The scope of protection is defined by the claims.

In a first aspect there is provided a medical device having irregular and/or curved silicone surfaces, wherein at least a part of the irregular and/or curved silicone surfaces is coated with rhamnolipids, wherein the medical device is a voice prosthesis (100) for mounting in a fistula between trachea and esophagus, comprising a tubular body (101) having a lumen, and a voice prosthesis valve comprising a valve disc (105) and a valve seat (106) comprising sealing surfaces (108), said valve disc (105) and said valve seat (106) arranged in the lumen of the tubular body (101) and controlling the communication through said lumen by interaction between said valve disc (105) and said valve seat (106), wherein at least one of the sealing surfaces (108) of the voice prosthesis valve and/or at least one of the retaining flanges (102, 103) are silicone surfaces coated with rhamnolipids, wherein the rhamnolipids are covalently attached to the surfaces via a linker comprising an amide bond, wherein the linker is -O-Si-(CH2)3-N-CO- or -O- (CH2)3-N-CO-, and wherein the rhamnolipids comprise 70-85% (weight/weight) mono-rhamnolipids and 15-30% (weight/weight) di-rhamnolipids, wherein the total amount of mono-rhamnolipids and di-rhamnolipids is 100% (weight/weight), and wherein the rhamnolipids have a purity of at least 85%. Such a coating will prevent or reduce the formation of a biofilm on the surface of the medical device.

In a second aspect there is provided a method of coating a medical device having irregular and/or curved silicone surfaces, the medical device being the voice prosthesis of claims 1 or 2 with rhamnolipids, wherein the method comprises the steps of 1) activating the silicone surfaces that are to be coated by introducing free amino-groups; and 2) covalently attaching rhamnolipids to said silicone surfaces. Such a method will result in a stable coating of rhamnolipids on the surfaces of a medical device.

Other objectives, features and advantages of the present disclosure will appear from the following detailed description, from the drawings, as well as from the attached claims. It is noted that the disclosure relates to all possible combination of features.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

### Acronyms and Reference signs

- APTES: 3-Aminopropyltriethoxysilane
- BS: biosurfactant
- 100: voice prosthesis
- 101: tubular body
- 102: esophageal retention flange
- 103: tracheal retention flange
- 104: valve member
- 105: valve disc
- 106: a valve seat
- 107: valve hinge
- 108: sealing surfaces
- Silicone: not treated silicone
- Silicone_AC7: not treated silicone with AC7 physical adsorption
- Silicone_R89: not treated silicone with R89 physical adsorption
- Silicone/P: argon plasma treated silicone
- Silicone/P_AC7: argon plasma treated silicone with AC7 physical adsorption
- Silicone/P_R89: argon plasma treated silicone with R89 physical adsorption
- Silicone/P_A: argon plasma treated silicone with APTES polymerization
- Silicone/P_A_AC7: AC7 covalent grafting on argon plasma treated silicone with APTES polymerization
- Silicone/P_A_R89: R89 covalent grafting on argon plasma treated silicone with APTES polymerization

### Brief Description of the Drawings

These and other aspects, features and advantages of which the invention is capable of will be apparent and elucidated from the following description of embodiments of the present invention, reference being made to the accompanying drawings, in which:
Fig. 1a shows a surface functionalized with APTES.
Fig. 1b shows a surface functionalized with cyclopropylamine.
Fig. 2a shows a scheme of biosurfactant (BS) covalent grafting on a silicone surface.
Fig. 2b shows a scheme of biosurfactant (BS) covalent grafting on a silicone surface.
Fig. 3a shows ATR-FTIR spectra of untreated silicone elastomeric discs (SEDs) (upper curve) and SEDs coated with R89BS by physical adsorption (lower curve).
Fig. 3b shows the contact angle values of silicone (Silicone); untreated silicone with physically adsorbed AC7 (Silicone_AC7); and untreated silicone with physically adsorbed R89 (rhamnolipids).
Fig. 4a shows an ATR-FTIR spectrum of argon plasma treated silicone with R89 physical adsorption (Silicone/P_R89).
Fig. 4b shows the contact angle values silicone (Silicone); argon plasma treated silicone (Silicone/P); argon plasma treated silicone with AC7 physical adsorption (Silicone/P_AC7); and argon plasma treated silicone with R89 physical adsorption (Silicone/P_R89).
Fig. 4c shows SEM (scanning electron microscopy) images of surface samples of: silicone (Silicone), plasma treated silicone with adsorbed AC7 (Silicone/P_AC7), and plasma treated silicone with adsorbed R89 (rhamnolipids) (Silicone/P_R89).
Fig. 5a shows ATR-FTIR spectra of untreated silicone (Silicone); argon plasma treated silicone with APTES polymerization (Silicone/P_A); R89 covalently grafted on argon plasma treated silicone with APTES polymerization (Silicone/P_A_R89).
Fig. 5b shows an ATR-FTIR spectrum of AC7 covalently grafted on argon plasma treated silicone with APTES polymerization (Silicone/P_A_AC7).
Fig. 5c shows the contact angle values of silicone (Silicone), argon plasma treated silicone (Silicone/P), argon plasma treated silicone with APTES polymerization (Silicone/P_A_AC7), argon plasma treated silicone with APTES polymerization and covalently grafted AC7 (Silicone/P_A_AC7) and argon plasma treated silicone with APTES polymerization and covalently grafted R89 (Silicone/P_A_R89).
Fig. 5d shows the contact angle values of silicone (Silicone) and of argon plasma treated silicone (Silicone/P) after 1 to 5 hours.
Fig. 6 ATR-FTIR spectra of silicone (Silicone); argon plasma treated silicone with APTES polymerization (Silicone/P_A); argon plasma treated silicone with AC7 physical adsorption (Silicone/P_AC7).
Fig. 7a shows a voice prosthesis according to the present disclosure.
Fig. 7b shows a voice prosthesis according to the present disclosure mounted in a fistula between trachea and esophagus.

### Description of Embodiments

The present disclosure relates to a voice prosthesis having irregular and/or curved silicone surfaces, wherein at least a part of the irregular and/or curved silicone surfaces is coated with rhamnolipids. The present disclosure also relates to a method of coating a voice prosthesis having irregular and/or curved silicone surfaces with rhamnolipids.

Specific aspects and embodiments of the present disclosure will be described in detail below.

Microorganisms synthesize a wide range of surface-active compounds (SAC), generally referred to as biosurfactants (BS). Microbial biosurfactants function as anti-adhesives and prevent or reduce the formation of biofilms. In the present disclosure, rhamnolipids are used for coating medical devices. Rhamnolipids are able to significantly inhibit fungal and bacterial biofilm formation on silicone. Thus, rhamnolipids inhibit fungal and bacterial biofilm growth and can prolong the device lifetime of medical devices of silicone and potentially reduce the use of antimicrobial drugs.

The rhamnolipids of the present disclosure are composed of homologues of the mono- and di-rhamnolipid families. The rhamnolipids may be produced by the bacteria *Pseudomonas aeruginosa* 89 and such rhamnolipids are herein referred to as R89 or R89BS.

The purity of the raw extract is typically about 91%. Typically, the rhamnolipids comprise 70-85% (weight/weight), preferably 75% (weight/weight), mono-rhamnolipids and 15-30% (weight/weight), preferably 25% (weight/weight), di-rhamnolipid.

At pH 7.0, the surface tension of alkaline distilled water, measured with a ring tensiometer, decreases rapidly as the concentration of biosurfactant is increased, with a minimum surface tension of about 30 mN/m. The maximum surfactant concentration in a solution that can be achieved without formation of clusters of surfactant molecules, so called micelles, defines the Critical Micelle Concentration (CMC). The CMC value for R89BS has been found to be at a concentration of 40 to 50 µg/mL, such as 42 to 48 µg/mL, such as about 46.1 µg/mL. Thus, the CMC value indicates the amount of surfactant required to reach maximum surface tension reduction, and the lower the CMC, the less surfactant is required to reduce the surface tension. Biofilm formation is reduced at low surface tension. Hence, in order to accomplish a certain reduction of biofilm formation, a lower concentration of R89BS is needed than of a biosurfactant having a higher CMC. This is reflected in the rather low minimal inhibitory concentrations (MICs) of crude rhamnolipid. The MIC of crude rhamnolipid is 0.06 mg/ml for S. *aureus* and 0.12 mg/ml for S. *epidermidis* and > 2 mg/ml for C. *albicans.*

Specifically, rhamnolipid raw extract, also referred to as rhamnolipid crude extract, has the ability to inhibit S. *aureus* adhesion to silicone and its activity is not significantly different from that of the mono- and di-rhamnolipid purified fractions. This implies that these two fractions contribute equally to the anti-adhesive activity of the crude extract. Thus, the crude extract can be used directly for coating of silicone surfaces for the inhibition of microbial growth and biofilm formation, without any further purification steps. This is advantageous, since downstream operation costs typically represent about 50-80% of the total processing costs.

A further advantage of rhamnolipids is that these biosurfactants retain their surface activity over a wide pH-range.

Furthermore, R89 covalently grafted to silicone according to the present disclosure is able to inhibit biofilm formation of *C*. *albicans* and *S*. *aureus* at eight days of incubation of 85% and 82 %, respectively.

### Medical devices

The medical devices disclosed herein have irregular and/or curved silicone surfaces, wherein at least a part of the irregular and/or curved silicone surfaces is coated with rhamnolipids. The formation of biofilms on such devices is reduced compared to devices not having a coating of rhamnolipids. Furthermore, as demonstrated in the experiments below, coating with rhamnolipid shows a significant ability to attach to silicone elastomer surfaces and to counteract biofilm formation of silicone elastomers compared to for example AC7BS (a lipopeptide from *Bacillus subtilis* AC7).

General features applicable to devices disclosed herein as well as specific examples of such devices are described below.

The rhamnolipids comprise 70-85% (weight/weight), preferably 75% (weight/weight), mono-rhamnolipids and 15-30% (weight/weight), preferably 25% (weight/weight), di-rhamnolipids. The total amount of mono-rhamnolipids and di-rhamnolipids is 100% (weight/weight).

In one specific embodiment, the rhamnolipids comprise 75% (weight/weight) mono-rhamnolipids and 25% (weight/weight), di-rhamnolipids.

The rhamnolipids have a purity of at least 85%, more preferably a purity of at least 90%.

The rhamnolipids are covalently attached to at least a part of the irregular and/or curved silicone surfaces. One advantage of such a coating is that the rhamnolipid coating is stable for a longer period of time than a rhamnolipid coating that is not covalently attached to the silicone surface.

The rhamnolipids are covalently attached to the surface via the linker comprising an amide-bond.

In one embodiment, the linker is -O-Si-(CH₂)₃-N-CO-. Such a coating may be achieved by using (3-aminopropyl)triethoxysilane (APTES) monomers.

In one embodiment, the linker is -O-(CH₂)₃-N-CO-. Such a coating may be achieved by using cyclopropylamine monomers.

In one embodiment, the surface density of rhamnolipids is more than 4.5 × 10¹⁵ rhamnolipids per cm², such as more than 5 × 10¹⁵ rhamnolipids per cm², such as more than 6 × 10¹⁵ rhamnolipids per cm², such as more than 7 × 10¹⁵ rhamnolipids per cm², such as more than 8 × 10¹⁵ rhamnolipids per cm². Such surfaces are particularly resistant to the formation of biofilms.

Preferably, the surface density of rhamnolipids is more than 6 × 10¹⁵ rhamnolipids per cm².

Specifically,
the medical device is a voice prosthesis 100, preferably a silicone voice prosthesis, comprising a voice prosthesis valve, wherein at least one of the sealing surfaces 108 of the voice prosthesis valve and/or at least one of the retaining flanges 102, 103 are silicone surfaces coated with rhamnolipids. The rhamnolipids are covalently attached to the surfaces via linker comprising an amide bond. The linker is -O-Si-(CH₂)₃-N-CO- or -O-(CH₂)₃-N-CO-. The rhamnolipids comprise 70-85% (weight/weight), preferably 75% (weight/weight), mono-rhamnolipids and 15-30% (weight weight), preferably 25% (weight/weight), di-rhamnolipids. The total amount of mono-rhamnolipids and di-rhamnolipids is 100% (weight/weight). The rhamnolipids have a purity of at least 85%, more preferably a purity of at least 90%.

### Methods of coating a medical device

The methods disclosed herein are for coating a medical device having irregular and/or curved silicone surfaces with rhamnolipids, the medical device being a voice prosthesis as described above.

General features applicable to the method disclosed herein as well as specific examples of such methods are described below.

The method comprises the steps of 1) activating the silicone surfaces that are to be coated by introducing free amino-groups; and 2) covalently attaching rhamnolipids to said silicone surfaces.

As demonstrated in the experiments below, a coating with rhamnolipid shows a significant ability to attach to silicone elastomer surfaces and to counteract biofilm formation of silicone elastomers compared to for example AC7BS (a lipopeptide from *Bacillus subtilis* AC7).

In one embodiment of the method, step 1) comprises subjecting the surface that is to be coated to (3-aminopropyl)triethoxysilane (APTES) monomers or cyclopropylamine monomers. By using such a step, the rhamnolipids will be covalently attached to the silicone surface via a linker comprising an amide-bond. APTES linkers have been demonstrated (see Experimental Section below) to be a good strategy in order to covalently graft biosurfactants on a silicone surface. The attachment of the APTES molecules on the silicone surface allows to expose amino groups (-NH₂), that can be exploited to form an amide-bond with the carboxyl group (-COOH) of biosurfactants. The physico-chemical and biological results demonstrated the efficacy of APTES coating to covalently graft R89. Cyclopropylamine is an alternative monomer to obtain a polymeric linker, exposing NH₂, to graft biosurfactants.

In one embodiment of the method, step 1) comprises the steps of a) subjecting the silicone surface that is to be coated to atmospheric or vacuum plasma discharge; and b) subjecting the silicone surface that is to be coated to a solution of (3-aminopropyl)triethoxysilane (APTES) monomers or cyclopropylamine monomers. Atmospheric or vacuum argon plasma discharge enhances the surface functionalization process. Subjecting the silicone surface that is to be coated to atmospheric or vacuum plasma discharge before covalent grafting of rhamnolipids such as rhamnolipids results in a high amount of rhamnolipids attached to the surface.

Typically, such a process comprising the steps of a) subjecting the silicone surface that is to be coated to atmospheric or vacuum plasma discharge; and b) subjecting the silicone surface that is to be coated to a solution of (3-aminopropyl)triethoxysilane (APTES) monomers or cyclopropylamine monomers, yields a surface having more than 6 × 10¹⁵ amino-groups per cm², such as more than 7 × 10¹⁵ amino-groups per cm², such as more than 8 × 10¹⁵ amino-groups per cm². In turn, this leads to a high surface density of the rhamnolipids attached to the surface.

The atmospheric plasma discharge may for example be atmospheric plasma discharge of argon, oxygen or hydrogen. The atmospheric plasma discharge may be of a mixture of any of these. For example, the atmospheric plasma discharge may be of a mixture of oxygen and hydrogen plasma discharge. In one preferred embodiment, the atmospheric plasma discharge is an argon plasma discharge. The parameters for the atmospheric plasma discharge may be power 10 to 100 W, such as 20 to 90 W, such as 30 to 80 W, such as 40 to 70 W, such as 50 to 60W; flow rate 5 to 20 sccm (standard cubic centimetre per minute), such as 10 to 15 sccm; time 10 seconds (s) to 10 minutes (min), such as 20 s to 5 min, such as 30 s to 3 min; such as 1 to 2 minutes. The distance from the outlet of the plasma nozzle to the sample may be 0.5 to 2.5 cm, such as 1 to 2 cm, such as 1.2 to 1.8 cm, such as 1.4 to 1.6 cm, preferably 1.5 cm.

The process may be optimized by changing the plasma parameters (power, flow rate, time and distance).

One suitable combination of parameters for the atmospheric plasma discharge may be power 10 W, flow rate 20 sccm (standard cubic centimetre per minute), time 20s and distance 5 mm. A further suitable combination of parameters may be power 100 W, flow rate 15 sccm (standard cubic centimetre per minute), time 5 min. Another suitable combination of parameters may be e.g. power 20 W, flow rate 10 sccm (standard cubic centimetre per minute), time 20s and distance 15 mm.

The vacuum plasma discharge may for example be vacuum plasma discharge of argon, oxygen or hydrogen. The vacuum plasma discharge may be of a mixture of any of these. For example, the vacuum plasma discharge may be of a mixture of oxygen and hydrogen plasma discharge. In one preferred embodiment, the vacuum plasma discharge is an argon plasma discharge.

Preferably, the plasma discharge is vacuum plasma discharge.

The solution of (3-aminopropyl)triethoxysilane (APTES) monomers is a 2 to 10 % (volume/volume, preferably 3 to 7 % (volume/volume, such as 4 to 6 % (volume/volume), even more preferred a 5 % (volume/volume) APTES solution with ethanol as solvent.

The solution of cyclopropylamine monomers is a 2 to 10 % (volume/volume), preferably 3 to 7 % (volume/volume, such as 4 to 6 % (volume/volume), even more preferred a 5 % (volume/volume) cyclopropylamine solution with an organic solvent, such as ethanol.

The silicone surface that is to be coated may be subjected to the solution of (3-aminopropyl)triethoxysilane (APTES) monomers or cyclopropylamine monomers through immersion. The immersion may take place for 15 to 60 minutes, such as 20 to 40 minutes, preferably 30 minutes. Preferably, the immersion takes place at room temperature. After immersion, the silicone surface may be rinsed, preferably rinsed with ethanol.

The silicone surface that is to be coated may be subjected to the solution of (3-aminopropyl)triethoxysilane (APTES) monomers or cyclopropylamine monomers at room temperature.

Optionally, step b) above is followed by a step of subjecting the silicone surface that is to be coated to a temperature of 50 to 150 °C, such as 70 to 130 °C, such as 80 to 120 °C, preferably 90 to 110 °C, such as 95 to 105 °C, even more preferred around 100 °C, for a time period of 5 to 45 minutes, preferably, 10 to 30 minutes, such as 15 to 25 minutes, even more preferred around 20 minutes. Subjecting the silicone surface that is to be coated to a high temperature contributes to the stabilization of the APTES or cyclopropylamine coating.

In a specific embodiment, step 1) comprises the steps of a) subjecting the silicone surface that is to be coated to atmospheric or vacuum plasma discharge, preferably wherein the vacuum plasma discharge is performed using the following parameters: power 20 W, flow rate 10 sccm (standard cubic centimetre per minute), time 20s and distance 15 mm, followed b) subjecting the silicone surface that is to be coated to a solution of (3-aminopropyl)-triethoxysilane (APTES) monomers in ethanol, preferably 5% (vol/vol) APTES, for a time period of 30 minutes. Optionally, step b) is followed by a step of subjecting the silicone surface that is to be coated to a temperature of 50 to 150 °C, such as 70 to 130 °C, such as 80 to 120 °C, preferably 90 to 110 °C, such as 95 to 105 °C, even more preferred around 100 °C, for a time period of 5 to 45 minutes, preferably, 10 to 30 minutes, such as 15 to 25 minutes, even more preferred around 20 minutes. Preferably, the silicone surface is thereafter cured at around 100 °C, for a time period of 20 minutes.

Typically, such a process yields a surface having more than 6 × 10¹⁵ amino-groups per cm², such as more than 7 × 10¹⁵ amino-groups per cm², such as more than 8 × 10¹⁵ amino-groups per cm². In turn, this leads to a high surface density of the rhamnolipids attached to the surface.

According to another embodiment of the method, step 1) comprises the steps of: i) subjecting the silicone surface that is to be coated to atmospheric or vacuum plasma discharge of argon in combination with (3-aminopropyl)-triethoxysilane (APTES) monomers or of argon in combination with cyclopropylamine monomers. The monomers are directly polymerized on the silicone surface during the plasma treatment. This results in the generation of amino groups on the silicone surface by polymerizing monomers. Surface functionalization using plasma technique can be used to apply either APTES monomers or cyclopropylamine monomers. An advantage of using plasma discharge for this type of direct application of monomers creating the surface functionalization is that it increases the precision and allows selection of which surfaces to treat and what surfaces to leave out. Another advantage is that, by direct application of surface functionalization through plasma discharge, the amount of monomers can be controlled by adjustment of plasma discharge process parameters (e.g. discharge power, gas flow, process times). Furthermore, precise application can be achieved by optimizing the angle of the plasma stream to local variations in surface angle of irregular silicone surfaces. Suitable angles are between 0° and 30°, such as 10° to 20°, such as 15°. Further, the distance from the outlet of the plasma nozzle to the sample may be 0.5 to 2.5 cm, such as 1 to 2 cm, such as 1.2 to 1.8 cm, such as 1.4 to 1.6 cm, preferably 1.5 cm.

Further parameters for the atmospheric plasma discharge may be power 10 to 100 W, such as 20 to 90 W, such as 30 to 80 W, such as 40 to 70 W, such as 50 to 60W; flow rate 5 to 20 sccm (standard cubic centimetre per minute), such as 10 to 15 sccm; time 10 seconds (s) to 10 minutes (min), such as 20 s to 5 min, such as 30 s to 3 min; such as 1 to 2 minutes.

The process may be optimized by changing the plasma parameters (power, flow rate, time and distance).

One suitable combination of parameters for the atmospheric plasma discharge may be power 10 W, flow rate 20 sccm (standard cubic centimetre per minute), time 20s and distance 5 mm. A further suitable combination of parameters may be power 100 W, flow rate 15 sccm (standard cubic centimetre per minute), time 5 min. Another suitable combination of parameters may be e.g. power 20 W, flow rate 10 sccm (standard cubic centimetre per minute), time 20s and distance 15 mm.

Furthermore, a method as described above allowing for the polymerization of aminopropyl)-triethoxysilane (APTES) monomers or of cyclopropylamine monomers on the silicone surface is less time-consuming and more reproducible. In addition, the method is solvent-free. Furthermore, such a method is scalable to industry level. The monomer may be in a carrier gas. The carrier gas may be O₂ or H₂.

Optionally, step i) above is followed by a step of subjecting the silicone surface that is to be coated to a temperature of 50 to 150 °C, such as 70 to 130 °C, such as 80 to 120 °C, preferably 90 to 110 °C, such as 95 to 105 °C, even more preferred around 100 °C, for a time period of 5 to 45 minutes, preferably 10 to 30 minutes, such as 15 to 25 minutes, even more preferred around 20 minutes. Subjecting the silicone surface that is to be coated to a high temperature contributes to the stabilization of the APTES or cyclopropylamine coating.

In another specific embodiment, step 1) comprises the steps of a) subjecting the silicone surface that is to be coated to vacuum plasma discharge, preferably wherein the vacuum plasma discharge is performed using the following parameters: power 20 W, flow rate 30 sccm (standard cubic centimetre per minute), time 1 minute and distance 15 mm, followed by b) subjecting the silicone surface that is to be coated to a vapour of aminopropyl)-triethoxysilane (APTES) monomers in the vacuum chamber via a vacuum plasma discharge. The resulting plasma polymerization of APTES uses plasma sources to generate a gas discharge that provides energy to activate or fragment gaseous or liquid monomers in order to initiate polymerization. The amount of monomers can be controlled by adjustment of the process parameters. Further, compared to the method described above in which the surface to be coated is subjected to a solution of (3-aminopropyl)triethoxysilane (APTES) monomers in ethanol for a period of time, this method is more precise and less time-consuming. Thus, this process is suitable for series production, since it is more scalable to industry level. Moreover, this method does not necessitate the use of any solvents, which produce potentially hazardous chemical waste.

The APTES monomer vapours may be obtained by heating liquid APTES monomer close to the boiling point.

Preferably, the APTES monomer vapours are obtained by flushing argon gas through liquid APTES monomer.

Preferably, step 2) is performed using the following parameters: 20 W, 30 sccm argon flow-rate and 20 sccm APTES monomer vapours flow-rate, 1 minute.

Typically, such a process, especially when the APTES monomer vapours are obtained by flushing argon gas through liquid APTES monomer, yields a surface having more than 6 × 10¹⁵ amino-groups per cm², such as more than 7 × 10¹⁵ amino-groups per cm², such as more than 8 × 10¹⁵ amino-groups per cm². In turn, this leads to a high surface density of the rhamnolipids attached to the surface.

Thus, in some embodiments, after step 1), the surface density of amino-groups is more than 6 × 10¹⁵ amino-groups per cm², such as more than 7 × 10¹⁵ amino-groups per cm², such as more than 8 × 10¹⁵ amino-groups per cm².

Step 2 above is performed using carbodiimide chemistry. An advantage of this method is a stronger and more stable attachment of biosurfactant compared to coating through physical adsorption.

Step 2 comprises the steps of 2a) mixing an aqueous solution of rhamnolipids with an aqueous solution comprising N-ethyl-N'-(3-dimethylaminopropyl) carbodiimide (EDC) and N-hydroxysuccinimide (NHS) at pH 4.5 to 6, preferably pH 5.5; 2b) increasing the pH to neutral pH, such as pH 7 to 7.5, preferably around pH 7.3 to 7.4, even more preferred pH 7.4 by the addition of NaOH; and 2c) subjecting the silicone surface that is to be coated to the mixture obtained in step 2b) for 1 to 24 h, preferably for 8 to 16 h, more preferred 10 to 14 h, such as around 12 h, at a temperature of 4 to 25 °C.

In 2a) above, the ratio of rhamnolipid solution to the aqueous solution containing N-ethyl-N'-(3-dimethylaminopropyl) carbodiimide and N-hydroxysuccinimide may be between 1:1 to 10:1, such as 2:1 to 9:1, such as 3:1 to 8:1, such as 4:1 to 7:1, such as 6:1 to 5:1. Preferably, the ratio is 2:1 to 6:1, such as 3:1 to 5:1. In one preferred embodiment, the ratio is 4:1.

The step of mixing 2a) may be performed for 1 hour at 4°C.

The ratio of EDC to NHS in the aqueous solution used in step 2a) may be from 2:1 to 10:1, preferably around 4:1. A ratio of more than 1:1 is especially advantageous since this accelerates the reaction.

The aqueous solution of rhamnolipids may for example be a 4 mg/mL solution.

The NaOH added in step 2b) is preferably 1N NaOH.

For optimization of the covalent grafting process, the ratio between EDC and NHS and the concentration of rhamnolipids can be further optimized between 1:1 to 10:1, such as 2:1 to 9:1, such as 3:1 to 8:1, such as 4:1 to 7:1, such as 6:1 to 5:1. Preferably, the ratio is 2:1 to 6:1, such as 3:1 to 5:1. In one preferred embodiment, the ratio is 4:1.

Step 2c) can be carried out by immersing the silicone parts to be grafted with rhamnolipids, for example in 1 mL activated rhamnolipid solution, for 24h at 4°C.

The methods described above may further comprise the step of washing the coated silicone surface obtained after step 2). The step of washing can for example comprise the steps of 3) rinsing the coated silicone surface obtained after step 2) with distilled water; and 4) drying the coated silicone surface under vacuum at room temperature.

The rhamnolipids used in the methods described above comprise 70-85% (weight/weight), preferably 75% (weight/weight), mono-rhamnolipids and 15-30% (weight/weight), preferably 25% (weight/weight) di-rhamnolipids. The total amount of mono-rhamnolipids and di-rhamnolipids is 100% (weight/weight).

In one specific embodiment, the rhamnolipids comprise 75% (weight/weight) mono-rhamnolipids and 25% (weight/weight), di-rhamnolipids.

The rhamnolipids have a purity of at least 85%, more preferably a purity of at least 90%.

In the method, the medical device is a voice prosthesis; wherein the medical device is a silicone voice 100 prosthesis comprising a voice prosthesis valve, wherein the surface coated with rhamnolipids comprise at least one of the sealing surfaces 108 of the voice prosthesis valve and/or at least one of the retaining flanges 102, 103 of the voice prosthesis. Coating at least one of the retaining flanges 102, 103 may prevent microbial growth that affect the strength of the retaining flanges negatively.

In another specific embodiment of the method, the medical device is a silicone voice prosthesis, wherein at least one of the retaining flanges of the voice prosthesis are coated with rhamnolipids. The rhamnolipids are covalently attached to the surfaces via linker comprising an amide bond. The linker may be -O-Si-(CH₂)₃-N-CO- or -O-(CH₂)₃-N-CO-. The rhamnolipids comprise 70-85% (weight/weight), preferably 75% (weight/weight), mono-rhamnolipids and 15-30% (weight weight), preferably 25% (weight/weight), di-rhamnolipids. The total amount of mono-rhamnolipids and di-rhamnolipids is 100% (weight/weight). The rhamnolipids have a purity of at least 85%, more preferably a purity of at least 90%. Coating one or more of the retaining flanges can prevent microbial growth affecting the strength of the retaining flanges negatively.

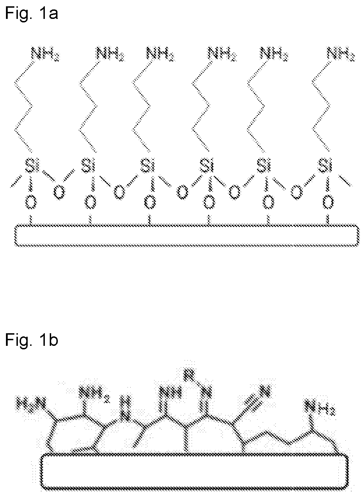

Fig 1a shows a surface functionalized with APTES.

Fig 1b shows a surface functionalized with cyclopropylamine.

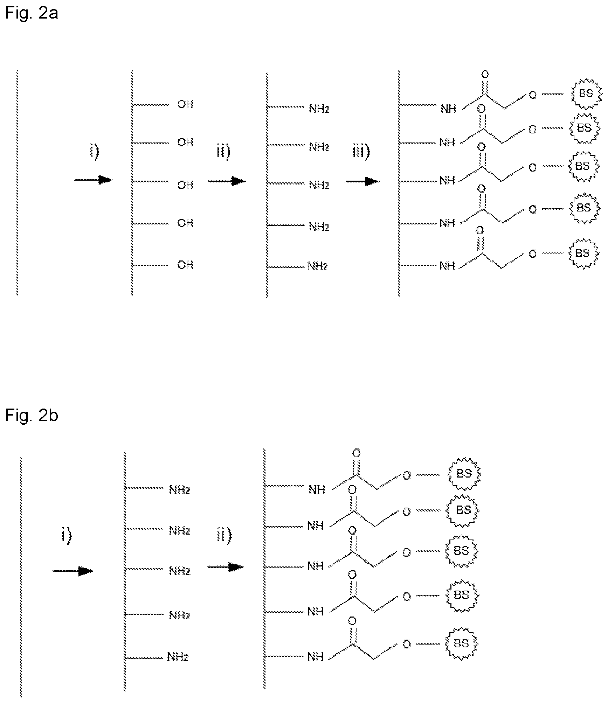

A specific method according to the present disclosure is shown in Fig 2a. In step (i) the silicone surface is subjected to argon plasma; in step (ii) the surface is subjected to a APTES monomer or a cyclopropylamine monomer to functionalize the surface; and in step (iii) the rhamnolipids are covalent grafted to the surface via carbodiimide chemistry with EDC/NHS. Step ii) is preferably carried out by immersing the silicone surface in a solution of the monomer, preferably 5% (volume/volume) in ethanol, for 20 to 40 minutes, preferably for 30 minutes, at room temperature. The silicone surface may thereafter be rinsed, for example with ethanol, and cured at 50 to 150 °C, such as 70 to 130 °C, such as 80 to 120 °C, preferably 90 to 110 °C, such as 95 to 105 °C, even more preferred around 100 °C, for a time period of 5 to 45 minutes, preferably, 10 to 30 minutes, such as 15 to 25 minutes, even more preferred around 20 minutes. Preferably, the silicone surface is cured at 100 °C, for a time period of 20 minutes. Preferably, step iii) comprises mixing equal volumes of an aqueous solution (4 mg/mL) of rhamnolipids with an aqueous solution containing N-ethyl-N'-(3-dimethylaminopropyl) carbodiimide (EDC)/N-hydroxysuccinimide (NHS) (4:1) (pH 5.5) for 1h at 4 °C; raising the pH to 7.4 by adding drops of 1N NaOH; followed by subjecting the silicone surfaces obtained in step i) to the solution obtained in step ii) for 20 to 30 h, preferably 24 hours, at 2 to 8 °C, preferably 5 °C, followed by rinsing with distilled water and drying, preferably drying under vacuum for 10 15 h, preferably for 12 h, at room temperature.

Another specific method according to the present disclosure is shown in Fig. 2b. The surface is functionalized (i) via plasma treatment with APTES monomer or cyclopropylamine; followed by (ii) rhamnolipid covalent grafting of rhamnolipids via carbodiimide chemistry with EDC/NHS. Preferably, step i) is carried out by subjecting the silicone surface that is to be coated to atmospheric or vacuum plasma discharge of argon in combination with (3-aminopropyl)-triethoxysilane (APTES) monomers or of argon in combination with cyclopropylamine monomers. The silicone surface may thereafter be rinsed, for example with ethanol, and cured at 50 to 150 °C, such as 70 to 130°C, such as 80 to 120 °C, preferably 90 to 110 °C, such as 95 to 105 °C, even more preferred around 100 °C, for a time period of 5 to 45 minutes, preferably, 10 to 30 minutes, such as 15 to 25 minutes, even more preferred around 20 minutes. Preferably, the silicone surface is cured at 100 °C, for a time period of 20 minutes. Preferably, step iii) comprises mixing equal volumes of an aqueous solution (4 mg/mL) of rhamnolipids with an aqueous solution containing N-ethyl-N'-(3-dimethylaminopropyl) carbodiimide (EDC)/N-hydroxysuccinimide (NHS) (4/1) (pH 5.5) for 1h at 4 °C; raising the pH to 7.4 by adding drops of 1N NaOH; followed by subjecting the silicone surfaces obtained in step i) to the solution obtained in step ii) for 20 to 30 h, preferably 24 hours, at 2 to 8 °C, preferably 5 °C, followed by rinsing with distilled water and drying, preferably drying under vacuum for 10 to 15 h, preferably for 12 h, at room temperature.

### Voice prosthesis

General features applicable to the vice prosthesis disclosed herein as well as specific examples of such voice prostheses are described below.

Disclosed herein is a voice prosthesis for mounting in a fistula between trachea and esophagus, wherein at least a part of the voice prosthesis 100 comprises silicone and at least a part of said silicone being coated with rhamnolipids. The voice prosthesis is for mounting in a fistula or puncture of the tracheoesophageal party wall P.

The voice prosthesis 100 for mounting in a fistula between trachea T and esophagus E, comprises a tubular body 101 having a lumen; a valve disc 105 and a valve seat 106, arranged in the lumen of the tubular body 101, said valve disc 105 and said valve seat 106 controlling the communication through said lumen by interaction between said valve disc 105 and said valve seat 106; wherein at least a part of the voice prosthesis 100 comprises silicone and at least a part of said silicone being coated with rhamnolipids.

The rhamnolipids comprise 70-85% (weight/weight), preferably 75% (weight/weight), mono-rhamnolipids and 15-30% (weight/weight), preferably 25% (weight/weight), di-rhamnolipids. The total amount of mono-rhamnolipids and di-rhamnolipids is 100% (weight/weight). Preferably, the rhamnolipids comprise 75% (weight/weight) mono-rhamnolipids and 25% (weight/weight), di-rhamnolipids.

The rhamnolipids have a purity of at least 85%, more preferably a purity of at least 90%.

The rhamnolipids are covalently attached to at least a part of the irregular and/or curved silicone surfaces. One advantage of such a coating is that the rhamnolipid coating is stable for a longer period of time than a rhamnolipid coating that is not covalently attached to the silicone surface.

The rhamnolipids are covalently attached to the surface via a linker comprising an amide-bond.

The linker is -O-Si-(CH₂)₃-N-CO- or -O-(CH₂)₃-N-CO-.

In one embodiment, the tubular body 101 comprises a distal and a proximal axial end, wherein the proximal end is provided with a esophageal retention flange 102 and the distal end is provided with a tracheal retention flange 103, and wherein at least a part of the tubular body 101, the esophageal retention flange 102 and/or the tracheal retention flange 103 comprises silicone and at least a part of said silicone being coated with rhamnolipids.

In another embodiment, the tubular body 101, the esophageal retention flange 102 and/or the tracheal retention flange 103 is made of silicone and said silicone being coated with rhamnolipids.

The valve disc 105 and/or the valve seat 106 comprises silicone and said silicone being coated with rhamnolipids.

In one specific embodiment, the tubular body 101 comprises a distal and a proximal axial end, wherein the proximal end is provided with a esophageal retention flange 102 and the distal end is provided with a tracheal retention flange 103, and wherein at least a part of the tubular body 101, the esophageal retention flange 102 and/or the tracheal retention flange 103 comprises silicone and at least a part of said silicone being coated with rhamnolipids. The rhamnolipids are covalently attached to the surface via a linker comprising an amide-bond. The linker is preferably -O-Si-(CH₂)₃-N-CO- or -O-(CH₂)₃-N-CO-.

In another specific embodiment, the tubular body 101, the esophageal retention flange 102 and/or the tracheal retention flange 103 is made of silicone and said silicone being coated with rhamnolipids. The rhamnolipids are covalently attached to the surface via a linker comprising an amide-bond. The linker is preferably -O-Si-(CH₂)₃-N-CO- or -O-(CH₂)₃-N-CO-.

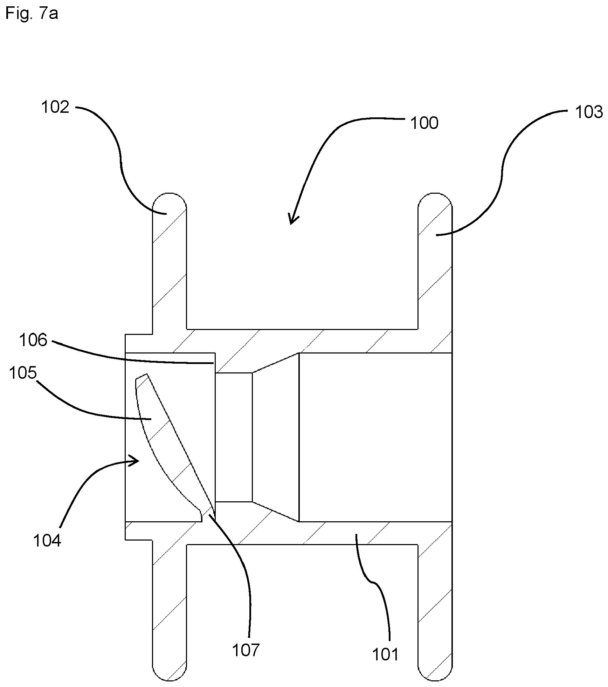

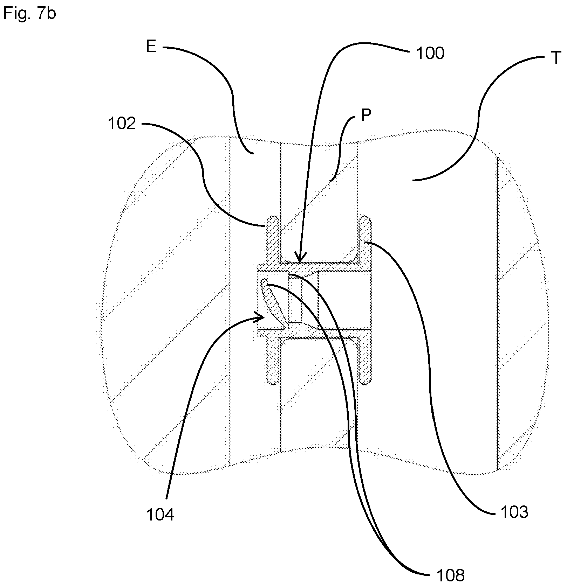

A voice prosthesis according to the present disclosure is shown in Fig. 7a. The voice prosthesis 100 comprises a tubular body 101. The tubular body 101 has a distal and a proximal axial end. The proximal end is intended to be facing the esophagus of the user, and the distal end is intended to be facing the trachea, during use (see Fig. 7b). The proximal end is provided with a esophageal retention flange 102. The distal end is provided with a tracheal retention flange 103. The esophageal and tracheal retention flanges 102 and 103 are arranged to extend laterally outwards from the tubular body 101. The retention flanges 102, 103 are arranged in a plane being transversal to a central axis of the tubular body 101. The tubular body 101 is manufactured in a flexible plastic or rubber material, such as silicone. When the tubular body 101 is manufactured in silicone, allergic reactions with the patient tissue may be decreased. Inside, i.e. in the lumen of the tubular body 101, a valve member 104 is arranged, said valve member 104 being suitable for closing the communication through the tubular body 101 but opens when subjected to airflow in the proximal direction. Airflow in the proximal direction is realized by the user when the user exhales at the same time as the user occludes the tracheostoma with his/her hand/finger or by activating a tracheostoma valve arranged to cover the user's tracheostoma.

The valve member 104 comprises a valve disc 105 and a valve seat 106. The valve disc 105 and the valve seat 106 are arranged in the tubular body 101. The valve disc 105 is arranged on a valve hinge 107, which in turn connects the valve disc 105 to the tubular body 101.

The valve disc 105 and/or the valve seat 106 may be manufactured in a rigid material resistant to bacterial and/or fungal growth, such as a fluorine polymer or a metal. An example of a suitable fluorine polymer is polyvinylidene difluoride (PVDF). Examples materials resistant to bacterial and/or fungal growth are stainless steel and titanium. Alternatively, the valve disc 105 and/or the valve seat 106 are made directly of silicone. The valve seat 106 is arranged circumferentially of the lumen of the valve body 107. The valve seat 106 may also be arranged in a plane that is traversing the lumen of the valve body 107. The valve hinge 107 may be manufactured in a suitable elastic material with good flexibility memory, such that the closing force will be adequately maintained for a long period of time. As suitable such elastic material is silicone.

Silicone surfaces on the voice prosthesis 100 may be coated with rhamnolipids. This is specifically beneficial, since the silicone surfaces on the voice prosthesis 100, such as the valve disc 105 and the valve seat 106 or the tubular body 101 are interacting during use to allow for a closing of the valve disc 105 against the valve seat 106.

Other silicone surfaces of the voice prosthesis 100 that may be coated with rhamnolipids as disclosed herein are one or more of the esophageal retention flange 102, the tracheal retention flange 103, and the at least one sealing surface 108 of the voice prosthesis valve.

The rhamnolipids, on the valve disc 105 and valve seat 106, respectively, especially on at least one of the sealing surfaces 108 of the voice prosthesis valve, will provide for a reduced risk of biofilm formation, which in turn reduces the risk of valve malfunction. However, additionally, the glycosyl head groups on the rhamnolipids, on the valve disc 105 and valve seat 106, respectively, especially on at least one of the sealing surfaces 108 of the voice prosthesis valve, have been found to reducing the risk for the valve disc 105 sticking to the valve seat 106, and therefore also reducing the risk for the valve member 104 to function improperly for this reason.

Fig. 7b shows a voice prosthesis according to the present disclosure mounted in a fistula between trachea and esophagus. The voice prosthesis is mounted in a fistula or puncture of the tracheoesophageal party wall P between the trachea T and the esophagus E. The esophageal retention flange 102 is located in the esophagus E and tracheal retention flange 103 is located in the trachea T.

Although the present invention has been described above with reference to specific embodiments, it is not intended to be limited to the specific form set forth herein. Rather, the invention is limited only by the accompanying claims and other embodiments than the specific embodiments described above are equally possible within the scope of these appended claims.

### Experimental Section

In the following, examples illustrating the devices and methods of the present disclosure are presented.

### Material and Methods

### Production of rhamnolipids (R89 also referred to as R89BS)

The bacteria *Pseudomonas aeruginosa* 89 is used as the biosurfactant producing strain, cultivated in Siegmund-Wagner (SW) medium, which is a mineral salt medium containing 0.7 g KH₂PO₄, 0.9 g, 2 g NaNOs, 0.4 g MgSO₄·7H₂O, 0.1 g CaCl₂·2H₂O and 2 mL of trace elements per 1000 mL distilled water. The trace element solution is prepared by dissolving 2 g FeSO₄·7H₂O, 1.5 g MnSO₄·H₂O and 0.6 g (NH₄)₆Mo₇O₂₄ in 1000 mL distilled water. In the final step the pH is adjusted to 6.7 and the medium is sterilized at 121°C for 15 minutes in an autoclave. Rhamnolipids (R89) are extracted from the acidified (pH 2.4 H₂SO₄, 6M) cell-free supernatant, using ethyl acetate. The organic phase is evaporated to dryness under vacuum conditions. The composition (i.e. the percentage of mono-rhamnolipids and di-rhamnolipids) of the rhamnolipid crude extracts can be confirmed with ESI-MS (Electrospray Ionization Mass Spectrometry) analysis.

### AC7 (also referred to as AC7BS)

AC7 (a biosurfactant, BS) was extracted from cultures *of Bacillus subtilis* AC7 and was used as a comparative compound in the examples below. AC7 is mainly composed of surfactin.

### Contact angle measurements

Static contact angle measurement was performed by depositing water drops with controlled volume in a controlled way.

In short, the static contact angle analysis was performed using the Attention Theta instrument (Biolin Scientific, Gothenburg, SE), equipped with automatic drop deposition system, and software OneAttension software (Biolin Scientific, Gothenburg, SE). Measurements were carried out in static conditions using water drops with 5 µL volume, at least 3 drops were deposited on each type of samples.

The static contact angle is herein also referred to as contact angle.

Presence of biosurfactants increases the wettability of the silicone surface and thereby lower the contact angle of the surface. High wettability results in difficulty for microbes to adhere to the surface due to the surface being wet and slippery, which is desirable, whereas hydrophobic surfaces enhance bacterial/microbial adherence.

### Microbiological evaluation

Silicone samples (silicone elastomeric discs, SEDs), uncoated or coated with biosurfactant as described herein, were submerged in 1 mL of S. *aureus* and *C. albicans* suspensions at the concentration of 1 x 10⁷ CFU/mL and incubated at 37 °C for 24, 48, and 72 h. S. *aureus* was suspended in Tryptic Soy Broth (TSB) supplemented with 1% (weight/volume) glucose. *C. albicans* was suspended in PBS (phosphate buffered saline) supplemented with 10% (volume/volume) fetal bovine serum.

For C. *albicans,* after 90 minutes of incubation, SEDs were transferred onto new plates containing fresh medium.

For S. *aureus* and *C. albicans,* every 24 h, SEDs were transferred onto new plates containing fresh media. The fresh medium for S. *aureus* was TSB and for C. *albicans* Yeast Nitrogen Base Dextrose (YNBD) broth supplemented with 10% (volume/volume) fetal bovine serum. At the end of the incubation period, the medium was removed, and SEDs were washed twice with PBS to remove non-adherent cells.

The inhibitory effect of the biosurfactants was studied after 24h, 48h and 72h by Crystal Violet staining (biomass) and MTT assay (metabolic activity) assessments.

### Experiments and Results

### A - Physical adsorption of rhamnolipids (not according to the claimed invention)

Silicone samples (silicone elastomeric discs, SEDs) were cleaned with ethanol and then immersed in a solution of biosurfactant (BS) (2 mg/mL) in PBS (phosphate buffered saline) at pH 7 for 24 h at 37 °C, at 180 rpm. The biosurfactant was R89BS or AC7. Control SEDs were immersed in 1 mL of PBS only. The BSs solutions were aspirated and SEDs were dried.

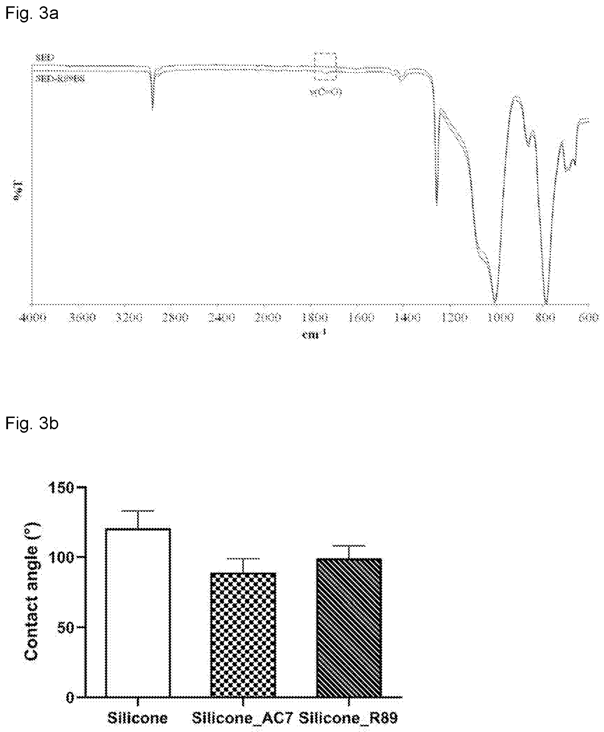

Fig. 3a shows ATR-FTIR spectra of untreated silicone elastomeric discs (SEDs) (upper curve) and SEDs coated with R89BS by physical adsorption (lower curve). As can be seen, in the FTIR-ATR spectrum of silicone coated with R89BS by physical adsorption, the appearance of the adsorption C=O band confirmed the presence of the R89 biosurfactant on silicone surface.

As can be seen in Table 1, a silicone disc coated with physically adsorbed rhamnolipids has a lower contact angle than an uncoated silicone disc. This decrease in contact angel confirms the presence of rhamnolipids since the rhamnolipids influence the wettability of the silicone.

**Table 1. Contact angle values of silicone elastomeric discs and silicone elastomeric discs coated with R89.**

| **Sample** | **Contact angle value (°)** |
|---|---|
| Silicone elastomeric discs | 112° ± 5° |
| Silicone elastomeric discs coated with R89 | 84.4° ± 2.2° |

Fig. 3b shows the contact angle values of silicone (Silicone); untreated silicone with physically adsorbed AC7 (Silicone_AC7); and untreated silicone with physically adsorbed R89 (rhamnolipids). In accordance with the results shown in Table 1, the contact angle value decreases when a biosurfactant is physically adsorbed to the untreated silicone. The contact angle value decreases more with AC7 than with R89.

The *in vitro* evaluation of the anti-biofilm activity of AC7 (a lipopeptide from *Bacillus subtilis* AC7) and R89 physically adsorbed on silicone elastomeric discs (SEDs) against biofilm-producer pathogenic strains (S. *aureus* ATCC 6538, C. *albicans* IHEM 2894) was performed as described above (microbiological evaluation).

The two biosurfactants were able to significantly counteract fungal biofilm formation. In particular, the highest biofilm inhibitory activity was observed with R89 at all incubation times both on biofilm biomass and on cells viability, with values respectively of 61% and 53% at 72h of incubation (Table 2a).

**Table 2a. Biomass and cell metabolic activity of C. albicans IHEM 2894 biofilms formed SEDs with biosurfactants deposited on the silicone surfaces by direct physical adsorption. Results are displayed with respect to uncoated controls.**

| **Time (h)** | **Assessment** | **CTRL** | | **AC7BS** | | **R89BS** | |
|---|---|---|---|---|---|---|---|
| | | **Mean** | **S.D.** | **Mean** | **S.D.** | **Mean** | **S.D.** |
| 24 | Biomass | 100 | 3 | 30 | 2 | 27 | 2 |
| | Metabolic activity | 100 | 7 | 35 | 4 | 29 | 3 |
| 48 | Biomass | 100 | 2 | 35 | 4 | 26 | 2 |
| | Metabolic activity | 100 | 5 | 58 | 3 | 37 | 3 |
| 72 | Biomass | 100 | 2 | 54 | 5 | 39 | 5 |
| | Metabolic activity | 100 | 4 | 64 | 6 | 47 | 3 |

A significant inhibition was observed on S. *aureus* biofilms with both biosurfactants. In particular, as also observed on *C. albicans,* R89 resulted to be the most active in S. *aureus* inhibition, with biofilm biomass and cells viability inhibitions of respectively 70% and 73% after 72h incubation (Table 2b).

Thus, even if AC7 gives a slightly lower contact angle value, the results from the biological evaluation as shown in Tables 2a and 2b clearly indicate that R89 (rhamnolipids) prevent the formation of a biofilm of both yeasts (*C. albicans* (Table 2a)) and bacteria (S. *aureus* (Table 2b)) to a larger degree than AC7.

**Table 2b. Biomass and cell metabolic activity of S. aureus ATCC 6538 biofilms formed on SEDs with biosurfactants deposited on the silicone surfaces by direct physical adsorption. Results are displayed with respect to uncoated controls.**

| **Time (h)** | **Assessment** | **CTRL** | | **AC7BS** | | **R89BS** | |
|---|---|---|---|---|---|---|---|
| | | **Mean** | **S.D.** | **Mean** | **S.D.** | **Mean** | **S.D.** |
| 24 | Biomass | 100 | 1 | 56 | 3 | 21 | 2 |
| | Metabolic activity | 100 | 2 | 55 | 4 | 23 | 4 |
| 48 | Biomass | 100 | 2 | 62 | 2 | 21 | 1 |
| | Metabolic activity | 100 | 4 | 66 | 5 | 20 | 4 |
| 72 | Biomass | 100 | 1 | 35 | 1 | 30 | 2 |
| | Metabolic activity | 100 | 5 | 32 | 2 | 27 | 3 |

### B - Plasma treated silicone samples followed by biosurfactant adsorption (not according to the claimed invention)

To generate reactive sites for subsequent adsorption of the biosurfactants, silicone discs were treated with argon plasma (5 min, flow 15 sccm (standard cubic centimetre per minute), power 100 W). Samples were then dipped into a 0.2% (weight/volume) AC7 or 0.2% (w/v) R89 aqueous solution at 37°C for 24h at 140 rpm.

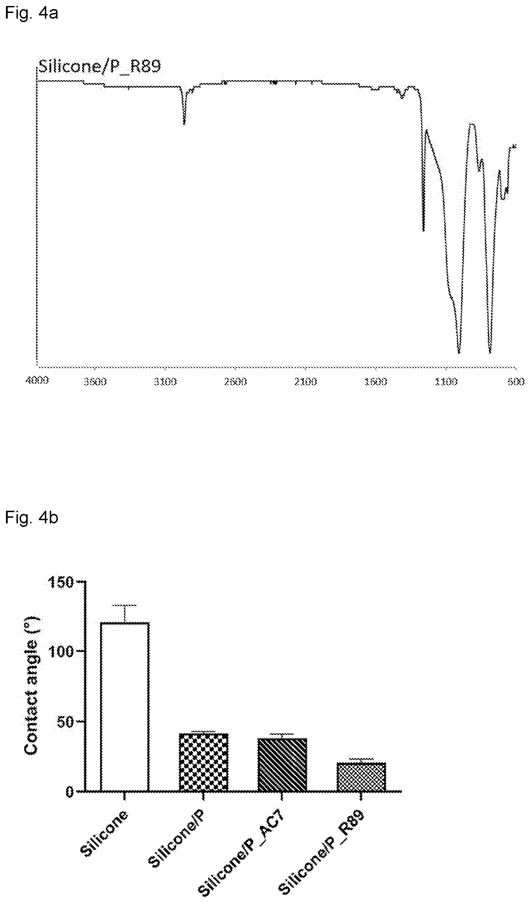

Fig. 4a shows an ATR-FTIR spectrum of argon plasma treated silicone with R89 physical adsorption (Silicone/P_R89). As can be seen, in the FTIR-ATR spectrum of plasma treated silicone coated with R89BS by physical adsorption, the appearance of the adsorption C=O band confirmed the presence of the R89 biosurfactants on silicone surface.

Fig. 4b shows the contact angle values silicone (Silicone); argon plasma treated silicone (Silicone/P); argon plasma treated silicone with AC7 physical adsorption (Silicone/P_AC7); and argon plasma treated silicone with R89 physical adsorption (Silicone/P_R89). The results clearly demonstrate that the plasma treatment decreases the contact angle value of the silicone, thus making the surface less prone to biofilm formation. Furthermore, the results presented in Fig. 4b show that the contact angle value of the silicone surface can be further decrease when a biosurfactant is physically adsorbed to the plasma treated silicone surface. Notably, this effect is slightly stronger for R89 (rhamnolipids) than for AC7 and the lowest contact angle value is thus achieved by plasma pre-treatment in combination with R89 adsorption. The results were measured just after preparing the surface. Over time, the contact angles increase for the control silicone samples without biosurfactant adsorption.

Compared to Experiment A (physical adsorption of biosurfactant only), a lower contact angle value is achieved by pretreating the silicone with plasma before coating with AC7 or R89. This implies that by pretreating the silicone surface with argon plasma, more biosurfactant is attached in the following step.

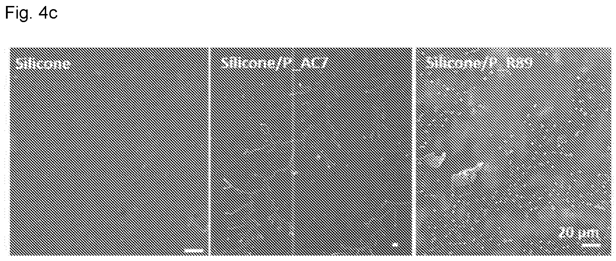

Fig. 4c show SEM (scanning electron microscopy) images of surface samples of: silicone (Silicone), plasma treated silicone with adsorbed AC7 Silicone/P_AC7), and plasma treated silicone with adsorbed R89 (rhamnolipids) (Silicone/P_R89). It is clearly seen that more R89 is attached to the silicone than AC7.

The *in vitro* evaluation of the anti-biofilm activity against biofilm-producer pathogenic strains (S. *aureus* ATCC 6538, *C. albicans* IHEM 2894) was performed as described above (microbiological evaluation).

Every 24 h, SEDs were transferred onto new plates containing fresh media. At the end of the incubation period, the growth medium was removed, and SEDs were washed twice with PBS to remove non-adherent cells.

As seen in Tables 3a and 3b, silicone/plasma-biosurfactant promoted a significant inhibition of biofilm formation for all the tested strains.

For *C*. *albicans* (Table 3a) the highest inhibition of biofilm formation was detected with R89 and the activity was stable at all incubation times. In particular, biomass and cell viability of fungal biofilm were respectively decreased of 71 % and 74 % at 72h incubation.

**Table 3a. Biomass and cell metabolic activity of C. albicans IHEM 2894 biofilms formed on biosurfactant- (BSs)-plasma SEDs. BSs physical adsorption was realized after the activation of silicone surfaces through argon plasma treatment. Results are displayed with respect to uncoated controls.**

| **Time (h)** | **Assessment** | **CTRL** | | **AC7BS** | | **R89BS** | |
|---|---|---|---|---|---|---|---|
| | | **Mean** | **S.D.** | **Mean** | **S.D.** | **Mean** | **S.D.** |
| 24 | Biomass | 100 | 2 | 50 | 2 | 28 | 1 |
| | Metabolic activity | 100 | 6 | 45 | 4 | 25 | 3 |
| 48 | Biomass | 100 | 3 | 56 | 1 | 29 | 2 |
| | Metabolic activity | 100 | 7 | 52 | 4 | 30 | 7 |
| 72 | Biomass | 100 | 4 | 57 | 3 | 29 | 3 |
| | Metabolic activity | 100 | 3 | 50 | 1 | 26 | 2 |

As far as *S*. *aureus* is concerned (Table 3b), the highest inhibition of biofilm formation was again detected with R89BS. In particular, biofilm biomass and cell viability were averagely inhibited of 86% at 72h incubation.

**Table 3b. Biomass and cell metabolic activity of S. aureus ATCC 6538 biofilms formed on BSs-plasma SEDs. BSs physical adsorption was realized after the activation of silicone surfaces through argon plasma treatment. Results are displayed with respect to uncoated controls.**

| **Time (h)** | **Assessment** | **CTRL** | | **AC7BS** | | **R89BS** | |
|---|---|---|---|---|---|---|---|
| | | **Mean** | **S.D.** | **Mean** | **S.D.** | **Mean** | **S.D.** |
| 24 | Biomass | 100 | 1 | 57 | 1 | 17 | 1 |
| | Metabolic activity | 100 | 7 | 63 | 8 | 12 | 2 |
| 48 | Biomass | 100 | 2 | 58 | 2 | 16 | 2 |
| | Metabolic activity | 100 | 3 | 70 | 4 | 12 | 2 |
| 72 | Biomass | 100 | 2 | 63 | 2 | 17 | 2 |
| | Metabolic activity | 100 | 4 | 73 | 3 | 11 | 2 |

Thus, the results from the biological evaluation as shown in Tables 3a and 3b clearly show that R89 (rhamnolipids) physically adsorbed to an argon plasma treated silicone surface prevent the formation of a biofilm of both yeasts (*C. albicans* (Table 2a)) and bacteria (S. *aureus* (Table 2b)) to a larger degree than AC7. This indicates that rhamnolipids are more effective than AC7 in preventing the formation of biofilms on silicone surfaces.

### C - Covalent grafting of biosurfactants on plasma treated silicone surfaces (not according to the claimed invention)

Silicone substrates pretreated with vacuum plasma discharge of argon were immersed in a solution of 3-aminopropyltriethoxysilane (APTES) in ethanol (5% v/v) for 30 min at room temperature, followed by rinsing with ethanol. Subsequently, samples were cured at 100°C for 20 min. The covalent grafting of BSs on silicone surfaces was obtained via carbodiimide chemistry. Briefly, 1 mL of biosurfactant aqueous solution (4 mg/mL) was mixed with 1 mL of an aqueous solution containing N-ethyl-N'-(3-dimethylaminopropyl) carbodiimide (EDC)/N-hydroxysuccinimide (NHS) (4/1) (pH 5.5) for 1h at 4 °C. Then, the pH was raised to 7.4 by adding drops of 1N NaOH. APTES- (or cyclopropylamine-) modified silicone substrates were immersed in 1 mL biosurfactant activated solution for 24h at 5°C and then washed three times using distilled water and dried under vacuum overnight at room temperature.

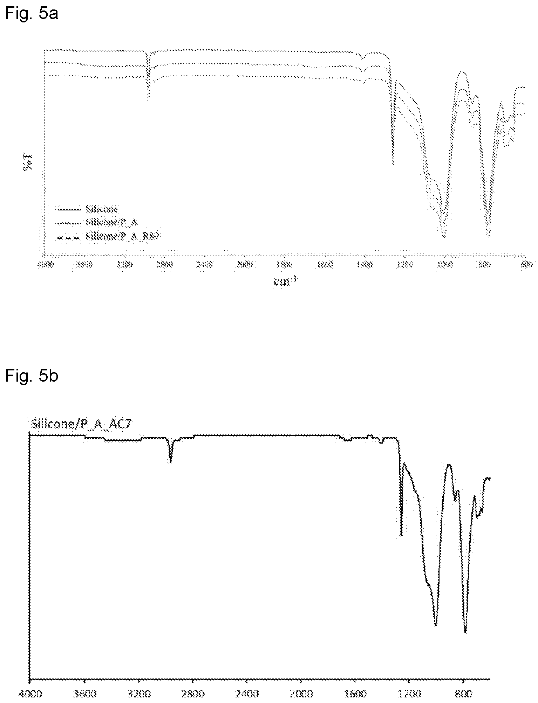

Fig. 5a shows ATR-FTIR spectra of untreated silicone (Silicone); argon plasma treated silicone with APTES polymerization (Silicone/P_A); R89 covalently grafted on argon plasma treated silicone with APTES polymerization (Silicone/P_A_R89). As can be seen, FTIR-ATR spectra of Silicone/P_A_R89 did not show any adsorption bands attributable to R89. This is probably caused by the sensitivity of ATR-FTIR instrument (2-5 µm): the covalent grafting allows lower amount of biosurfactant compared to the physical adsorption.

Fig. 5b shows an ATR-FTIR spectrum of AC7 covalently grafted on argon plasma treated silicone with APTES polymerization (Silicone/P_A_AC7). As can be seen, FTIR-ATR spectra of Silicone/P_A_AC7, did not show any adsorption bands attributable to AC7. This is probably caused by the sensitivity of ATR-FTIR instrument (2-5 µm): the covalent grafting allows lower amount of biosurfactant compared to the physical adsorption.

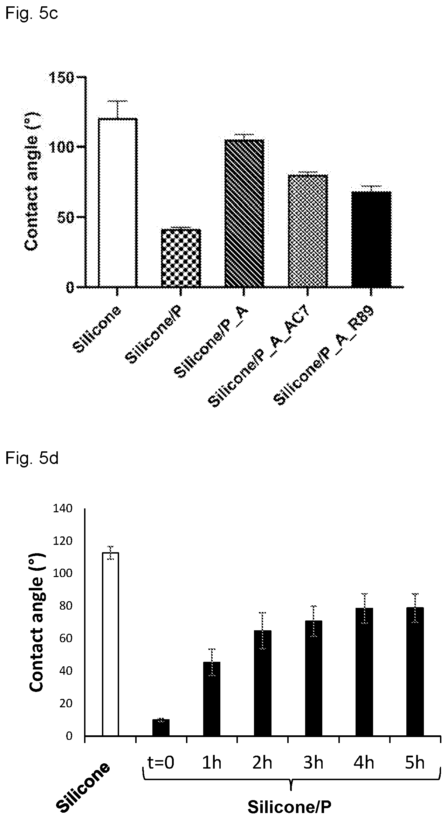

Fig. 5c shows the contact angle values of silicone (Silicone), argon plasma treated silicone (Silicone/P), argon plasma treated silicone with APTES polymerization (Silicone/P_A_AC7), argon plasma treated silicone with APTES polymerization and covalently grafted AC7 (Silicone/P_A_AC7) and argon plasma treated silicone with APTES polymerization and covalently grafted R89 (Silicone/P_A_R89). This data is also presented in Table 4a below.

While the contact angle value of plasma treated silicone samples (Silicone/P) is lowest, this result is not stable over time but increases within hours. This is shown in Fig. 5d. Thus after 4 to 5 hours, the static contact angle has increased to about 80°, which is higher than the contact angle for silicone treated with plasma and coated with R89 by physical adsorption or by covalent grafting.

**Table 4a. Contact angle values of silicone elastomeric discs and silicone elastomeric discs treated with plasma and coated with biosurfactants according to methods described herein.**

| **Sample** | **Contact angle value (°)** |
|---|---|
| Silicone | 120.6 ± 12.4 |
| Silicone_AC7 | 88.9 ± 9.9 |
| Silicone_R89 | 99.1 ± 8.8 |
| Silicone/P | 41.6 ± 1.3 |
| Silicone/P_AC7 | 38.1 ± 3.0 |
| Silicone/P_R89 | 20.6 ± 2.7 |
| Silicone/P_A | 105.2 ± 3.7 |
| Silicone/P_A_AC7 | 80.0 ± 2.0 |
| Silicone/P_A_R89 | 68.0 ± 4.0 |

Covalent grafting allows lower amounts of biosurfactant attached compared to physical adsorption. However, the attachment of the covalently grafted R89 is stronger and more stable, as can be seen as the contact angle value of the plasma treated silicone samples coated with APTES and R89 though covalent grafting is lower than that of the silicone samples coated with R89 through physical adsorption (see Fig. 3b). Furthermore, biosurfactant attached by covalent grafting, in this case by carbodiimide chemistry, are more stably attached over time than physically adsorbed biosurfactant. Thus, it has been clearly shown that covalent attachment of rhamnolipids to a plasma-treated silicone surface coated with APTES gives a stable coating that prevents the formation of biofilms on the silicone surface over time.

The *in vitro* evaluation of the anti-biofilm activity against biofilm-producer pathogenic strains (*S*. *aureus* ATCC 6538, *C. albicans* IHEM 2894) was performed as described above (microbiological evaluation).

**Table 4b. Biomass and cell metabolic activity of C. albicans IHEM 2894 biofilms formed on R89BS covalent grafting SEDs. Results are displayed with respect to uncoated controls.**

| **Time (h)** | **Assessment** | **CTRL** | | **R89BS** | |
|---|---|---|---|---|---|
| | | **Mean** | **S.D.** | **Mean** | **S.D.** |
| 24 | Biomass | 100 | 2 | 20 | 1 |
| | Metabolic activity | 100 | 4 | 25 | 2 |
| 48 | Biomass | 100 | 2 | 19 | 2 |
| | Metabolic activity | 100 | 2 | 24 | 4 |
| 72 | Biomass | 100 | 1 | 20 | 1 |
| | Metabolic activity | 100 | 2 | 22 | 1 |

As can be seen in Table 4b, the R89 covalent grafting on APTES modified silicone/P discs allowed to maintain the anti-biofilm activity up to 72 hours of incubation. *C. albicans* biofilm formation was reduced in term of biomass and metabolic activity by 80% and 78% respectively, at the last time-point (72h).

Biomass and metabolic activity of S. *aureus* biofilms were respectively reduced by 98% and 93% at 72h (see Table 4c).

The effect of these modified surfaces was constant, or even incremental, over time.

In conclusion, the results show a decreased biomass and cell metabolic activity on SEDs with covalently grafted R89 as compared to uncoated controls. Furthermore, the effect of reduced biofilm formation is larger than for silicone surfaces with physically adsorbed rhamnolipids (Tables 2a and 2b) and also for argon plasma treated silicone surfaces with physically adsorbed rhamnolipids (Tables 3a and 3b).

**Table 4c. Biomass and cell metabolic activity of S. aureus ATCC 6538 biofilms formed on R89 covalent grafting SEDs. Results are displayed with respect to uncoated controls.**

| **Time (h)** | **Assessment** | **CTRL** | | **R89** | |
|---|---|---|---|---|---|
| | | **Mean** | **S.D.** | **Mean** | **S.D.** |
| 24 | Biomass | 100 | 3 | 20 | 3 |
| | Metabolic activity | 100 | 4 | 24 | 5 |
| 48 | Biomass | 100 | 1 | 15 | 2 |
| | Metabolic activity | 100 | 5 | 11 | 3 |
| 72 | Biomass | 100 | 2 | 2 | 1 |
| | Metabolic activity | 100 | 5 | 7 | 2 |

Thus, it is clearly shown that a silicone surface being less prone to biofilm formation can be achieved by covalently grafting rhamnolipids on APTES-modified silicone surfaces pre-treated with argon plasma.

### D - Further comparative example

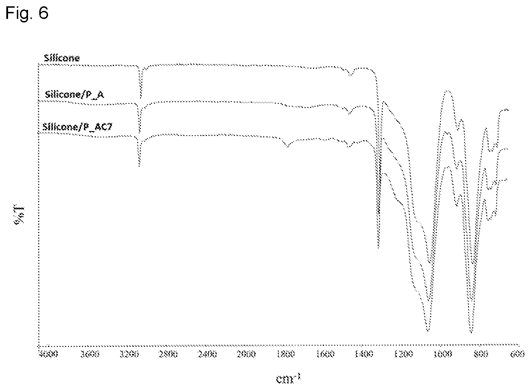

Fig. 6 ATR-FTIR spectra of silicone (Silicone); argon plasma treated silicone with APTES polymerization (Silicone/P_A); argon plasma treated silicone with AC7 physical adsorption (Silicone/P_AC7). As can be seen, ATR-FTIR analysis confirmed the polymerization of APTES on the silicone surface, whereas the adsorption band attributable to AC7 are not presented, due to the instrument sensitivity.

### E - Number of amino-groups on plasma treated silicone surfaces

The effect of different methods of introducing amino-groups on a silicone surface was investigated.

Test samples A were prepared by pretreating silicone samples by argon plasma discharge in a vacuum chamber (20W for 20 seconds) followed by immersion in a solution of (3-aminopropyl)triethoxysilane (APTES) monomers in ethanol (5% (vol/vol) APTES) for a time period of 30 minutes. Thereafter the samples were rinsed with ethanol and cured at 100 °C for a time period of 20 minutes, whereby the APTES coating was stabilized.

Test samples B were prepared by pretreating silicone samples with argon plasma discharge in a vacuum chamber, in continuous wave modality (20W for 1 minute). While still in the vacuum chamber, the silicone sample were subjected to APTES monomer vapours obtained by heating the liquid monomer close to the boiling point. Polymerisation of APTES monomers on the silicone surface was performed via a vacuum plasma discharge in the pulsed wave modality at 20 W, 30 sccm argon flow-rate and 20 ccm APTES monomer vapours flow-rate for 1 minute.

Test samples C were prepared by pretreating silicone samples with argon plasma discharge in a vacuum chamber, in continuous wave modality (20W for 1 minute). While still in the vacuum chamber, the silicone sample were subjected to APTES monomer vapours obtained by flushing argon gas through the liquid monomer making it "bubble". Polymerisation of APTES monomers on the silicone surface was performed via a vacuum plasma discharge in the pulsed wave modality at 20 W, 30 sccm argon flow-rate and 20 ccm APTES monomer vapours flow-rate for 1 minute.

The number of amino-groups on the surface of the silicon samples were measured by an Acid Orange assay. In short, the samples were immersed an acidic aqueous solution (pH = 3) of Acid Orange II (Formula I below) with concentration of 0.175 mg/mL overnight. During this time, the amino-groups present on the silicon surface bound to molecules of Acid Orange II (AO) with a 1:1 ratio.

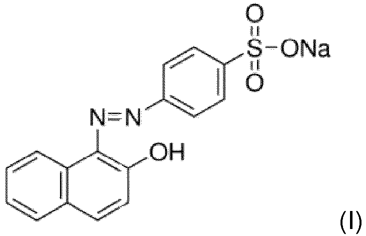

Samples were thereafter rinsed with pH 3 aqueous solution and immersed in a basic detachment solution (aqueous solution at pH = 12) for 30 minutes. In this way, all AO molecules that reacted with amino-groups were released into solution. For the quantification of AO, and therefore amino-groups, the detachment solution was sampled into a 96-well microtiter plate and absorbance was measured at 485 nm in Varioskan^{™} LUX multimode microplate reader. A calibration curve with different AO concentrations (ranging from 0.000313 mg/mL to 0.025 mg/mL) previously performed was used to quantify the amount of AO released from the samples and thereby the number of amino-groups groups present on the samples.

The results are shown in Table 5 below.

**Table 5. Number on amino-groups per cm² on silicon surfaces treated according to methods of the present disclosure. S.D. = Standard deviation**

| **Test samples** | **Number of amino-groups per cm²** | |
|---|---|---|
| | **Mean** | **S.D.** |
| A | 8.9 × 10¹⁵ | 1.5 × 10¹⁵ |
| B | 6.5 × 10¹⁵ | 0.91 × 10¹⁵ |
| C | 9.5 × 10¹⁵ | 0.84 × 10¹⁵ |

Samples A (in which the pretreated silicon surfaces were subjected to a solution of APTES in ethanol) and Samples C (in which the pretreated surfaces were subjected to APTES monomer vapours obtained by passing argon gas through the liquid monomer and a polymerization of the APTES monomers performed via a vacuum plasma discharge) yielded a higher density of amino-groups on the surface as compared to Samples B (in which the pretreated surfaces were subjected to APTES monomer vapours obtained by heating the liquid monomer close to the boiling point and a polymerization of the APTES monomers performed via a vacuum plasma discharge).

Thus, when rhamnolipids are covalently attached to surfaces treated as in Samples A, B or C, a high surface density of rhamnolipids is achieved. Specifically, when rhamnolipids are covalently attached to surfaces treated as in Samples A or C, a particularly high surface density of rhamnolipids is achieved.

## Claims

1. A medical device having irregular and/or curved silicone surfaces, wherein at least a part of the irregular and/or curved silicone surfaces is coated with rhamnolipids, wherein the medical device is a voice prosthesis (100) for mounting in a fistula between trachea and esophagus, comprising a tubular body (101) having a lumen, and a voice prosthesis valve comprising a valve disc (105) and a valve seat (106) comprising sealing surfaces (108), said valve disc (105) and said valve seat (106) arranged in the lumen of the tubular body (101) and controlling the communication through said lumen by interaction between said valve disc (105) and said valve seat (106), wherein at least one of the sealing surfaces (108) of the voice prosthesis valve and/or at least one of the retaining flanges (102, 103) are silicone surfaces coated with rhamnolipids, wherein the rhamnolipids are covalently attached to the surfaces via a linker comprising an amide bond, wherein the linker is -O-Si-(CH2)3-N-CO- or -O-(CH2)3-N-CO-, and wherein the rhamnolipids comprise 70-85% (weight/weight) mono-rhamnolipids and 15-30% (weight/weight) di-rhamnolipids, wherein the total amount of mono-rhamnolipids and di-rhamnolipids is 100% (weight/weight), and wherein the rhamnolipids have a purity of at least 85%.

2. A medical device according to claim 1, wherein the surface density of rhamnolipids is more than 4.5 × 10¹⁵ rhamnolipids per cm², such as more than 5 × 10¹⁵ rhamnolipids per cm², such as more than 6 × 10¹⁵ rhamnolipids per cm², such as more than 7 × 10¹⁵ rhamnolipids per cm², such as more than 8 × 10¹⁵ rhamnolipids per cm².

3. A method of coating a medical device having irregular and/or curved silicone surfaces, the medical device being the voice prosthesis of claims 1 or 2 with rhamnolipids, wherein the method comprises the steps of:
1) activating the silicone surfaces that are to be coated by introducing free amino-groups; and
2) covalently attaching rhamnolipids to said silicone surfaces.

4. The method according to claim 3, wherein step 1) comprises:
- subjecting the surface that is to be coated to (3-aminopropyl)-triethoxysilane (APTES) monomers or cyclopropylamine monomers.

5. The method according to claim 3 or 4, wherein step 1) comprises the steps of:
- subjecting the silicone surface that is to be coated to atmospheric or vacuum plasma discharge;
and
- subjecting the silicone surface that is to be coated to a solution of (3-aminopropyl)triethoxysilane (APTES) monomers or cyclopropylamine monomers;
and, optionally:
- subjecting the silicone surface that is to be coated to a temperature of 50 to 150 °C, such as 70 to 130 °C, such as 80 to 120 °C, preferably 90 to 110 °C, such as 95 to 105 °C, even more preferred around 100 °C, for a time period of 5 to 45 minutes, preferably 10 to 30 minutes, such as 15 to 25 minutes, even more preferred around 20 minutes.

6. The method according to claim 3 or 4, wherein step 1) comprises the steps of:
- subjecting the silicone surface that is to be coated to atmospheric or vacuum plasma discharge of argon in combination with (3-aminopropyl)triethoxysilane (APTES) monomers or of argon in combination with cyclopropylamine monomers;
and, optionally:
- subjecting the silicone surface that is to be coated to a temperature of 90 to 110 °C, such as 95 to 105 °C, even more preferred around 100 °C, for a time period of 5 to 45 minutes, preferably 10 to 30 minutes, such as 15 to 25 minutes, even more preferred around 20 minutes.

7. The method according to any one of claims 3 to 6, wherein, after step 1), the surface density of amino-groups is more than 6 × 10¹⁵ amino-groups per cm², such as more than 7 × 10¹⁵ amino-groups per cm², such as more than 8 × 10¹⁵ amino-groups per cm².

8. The method according to any one of claims 3 to 7, wherein step 2) is performed using carbodiimide chemistry.

9. The method according to any one of claims 3 to 8, wherein step 2 comprises the steps of:
2a) mixing an aqueous solution of rhamnolipids with an aqueous solution comprising N-ethyl-N'-(3-dimethylaminopropyl) carbodiimide and N-hydroxysuccinimide at pH 4.5 to 6, preferably pH 5.5;
2b) increasing the pH to 7.4 by the addition of NaOH;
and
2c) subjecting the silicone surface that is to be coated to the mixture obtained in step 2b) for 1 to 24 h, preferably for 8 to 16 h, more preferred 10 to 14h, such as around 12 h, at a temperature of 4 to 25°C.

10. The method according to any one of claims 3 to 9, further comprising the step of:
3) rinsing the coated silicone surface obtained after step 2) with distilled water;
and
4) drying the coated silicone surface under vacuum at room temperature.

11. The voice prosthesis (100) according to claim 1 or 2, wherein the tubular body (101) comprises a distal and a proximal axial end, wherein the proximal end is provided with a esophageal retention flange (102) and the distal end is provided with a tracheal retention flange (103), and wherein at least a part of the tubular body (101), the esophageal retention flange (102) and/or the tracheal retention flange (103) comprises silicone and at least a part of said silicone being coated with rhamnolipids.

12. The voice prosthesis (100) according to claim 11, wherein the tubular body (101), the esophageal retention flange (102) and/or the tracheal retention flange (103) is made of silicone and said silicone being coated with rhamnolipids.

## Patentansprüche

1. Medizinische Vorrichtung, die ungleichmäßige und/oder gekrümmte Silikonflächen aufweist, wobei mindestens ein Abschnitt der ungleichmäßigen und/oder gekrümmten Silikonflächen mit Rhamnolipiden beschichtet ist, wobei es sich bei der medizinischen Vorrichtung um eine Stimmprothese (100) zum Anbringen in einer Fistel zwischen Luftröhre und Speiseröhre handelt, wobei sie eine röhrenförmigen Körper (101) mit einem Hohlraum sowie ein Stimmprothesenventil umfasst, das eine Ventilscheibe (105) und einen Ventilsitz (106) umfasst, wobei diese abdichtende Flächen (108) umfassen, wobei die Ventilscheibe (105) und der Ventilsitz (106) in dem Hohlraum des röhrenförmigen Körpers (101) angeordnet sind und die Verbindung durch den Hohlraum mittels einer Wechselwirkung zwischen der Ventilscheibe (105) und dem Ventilsitz (106) steuern, wobei es sich bei mindestens einer der abdichtenden Flächen (108) des Stimmprothesenventils und/oder mindestens einem der Halteflansche (102, 103) um Silikonflächen handelt, die mit Rhamnolipiden beschichtet sind, wobei die Rhamnolipide über einen Linker, welcher eine Amidbindung umfasst, kovalent an die Flächen gebunden sind, wobei es sich bei dem Linker um -O-Si-(CH2)3-N-CO- oder -O-(CH2)3-N-CO- handelt und wobei die Rhamnolipide 70 bis 85 % (Gewicht/Gewicht) an Monorhamnolipiden und 15 bis 30 % (Gewicht/Gewicht) an Dirhamnolipiden umfassen, wobei die Gesamtmenge an Monorhamnolipiden und Dirhamnolipiden gleich 100 % (Gewicht/Gewicht) ist, und wobei die Rhamnolipide einen Reinheitsgrad von mindestens 85 % aufweisen.

2. Medizinische Vorrichtung gemäß Anspruch 1, wobei die Flächendichte der Rhamnolipide mehr als 4,5 × 10¹⁵ Rhamnolipide pro cm², wie etwa mehr als 5 × 10¹⁵ Rhamnolipide pro cm², wie etwa mehr als 6 × 10¹⁵ Rhamnolipide pro cm², wie etwa mehr als 7 × 10¹⁵ Rhamnolipide pro cm², wie etwa mehr als 8 × 10¹⁵ Rhamnolipide pro cm² beträgt.

3. Verfahren zum Beschichten einer medizinischen Vorrichtung, die ungleichmäßige und/oder gekrümmte Silikonflächen aufweist, wobei es sich bei der medizinischen Vorrichtung um eine Stimmprothese nach Anspruch 1 oder 2 handelt, mit Rhamnolipiden, wobei das Verfahren die folgenden Schritte umfasst:
1) Aktivieren der Silikonflächen, die beschichtet werden sollen, indem freie Aminogruppen eingeführt werden;
und
2) kovalentes Binden von Rhamnolipiden an die Silikonflächen.

4. Verfahren gemäß Anspruch 3, wobei der Schritt 1) Folgendes umfasst:
- Einwirkenlassen von (3-Aminopropyl)triethoxysilan(APTES)-Monomeren oder Cyclopropylamin-Monomeren auf die zu beschichtende Fläche.

5. Verfahren gemäß Anspruch 3 oder 4, wobei der Schritt 1) die folgenden Schritte umfasst:
- Einwirkenlassen von Plasmaentladungen bei Atmosphärendruck oder im Vakuum auf die zu beschichtende Silikonfläche;
und
- Einwirkenlassen von einer Lösung von (3-Aminopropyl)triethoxysilan(APTES)-Monomeren oder Cyclopropylamin-Monomeren auf die zu beschichtende Silikonfläche;
und, gegebenenfalls:
- Einwirkenlassen einer Temperatur von 50 bis 150 °C, wie etwa 70 bis 130 °C, wie etwa 80 bis 120 °C, vorzugsweise 90 bis 110 °C, wie etwa 95 bis 105 °C, noch stärker bevorzugt ungefähr 100 °C auf die zu beschichtende Silikonfläche, für eine Zeitdauer von 5 bis 45 Minuten, vorzugsweise 10 bis 30 Minuten, wie etwa 15 bis 25 Minuten, noch stärker bevorzugt ungefähr 20 Minuten.

6. Verfahren gemäß Anspruch 3 oder 4, wobei der Schritt 1) die folgenden Schritte umfasst:
- Einwirkenlassen einer Plasmaentladung von Argon bei Atmosphärendruck oder im Vakuum in Kombination mit (3-Aminopropyl)triethoxysilan(APTES)-Monomeren oder von Argon in Kombination mit Cyclopropylamin-Monomeren auf die zu beschichtende Silikonfläche;
und, gegebenenfalls:
- Einwirkenlassen einer Temperatur von 90 bis 110 °C, wie etwa 95 bis 105 °C, noch stärker bevorzugt ungefähr 100 °C auf die zu beschichtende Silikonfläche, für eine Zeitdauer von 5 bis 45 Minuten, vorzugsweise 10 bis 30 Minuten, wie etwa 15 bis 25 Minuten, noch stärker bevorzugt ungefähr 20 Minuten.

7. Verfahren gemäß einem beliebigen der Ansprüche 3 bis 6, wobei nach dem Schritt 1) die Flächendichte an Aminogruppen mehr als 6 × 10¹⁵ Aminogruppen pro cm², wie etwa mehr als 7 × 10¹⁵ Aminogruppen pro cm², wie etwa 8 × 10¹⁵ Aminogruppen pro cm² beträgt.

8. Verfahren gemäß einem beliebigen der Ansprüche 3 bis 7, wobei Schritt 2) unter Anwendung von Carbodiimid-Chemie durchgeführt wird.

9. Verfahren gemäß einem beliebigen der Ansprüche 3 bis 8, wobei der Schritt 2) die folgenden Schritte umfasst:
2a) Mischen einer wässrigen Lösung von Rhamnolipiden mit einer wässrigen Lösung, die N-Ethyl-N'-(3-dimethylaminopropyl)carbodiimid und N-Hydroxysuccinimid umfasst, bei einem pH-Wert von 4,5 bis 6, vorzugsweise von 5,5;
2b) Erhöhen des pH-Werts auf 7,4 durch Zusatz von NaOH; und
2c) Einwirkenlassen der Mischung, welche im Schritt 2b) erhalten wurde, auf die zu beschichtende Silikonfläche für 1 bis 24 Std., vorzugsweise für 8 bis 16 Std., insbesondere 10 bis 14 Std., wie etwa ungefähr 12 Std., bei einer Temperatur von 4 bis 25 °C.

10. Verfahren gemäß einem beliebigen der Ansprüche 3 bis 9, wobei es weiterhin die folgenden Schritte umfasst:
3) Spülen der beschichteten Silikonfläche, welche nach dem Schritt 2) erhalten wurde, mit destilliertem Wasser; und
4) Trocknen der beschichteten Silikonfläche unter Vakuum bei Raumtemperatur.

11. Stimmprothese (100) gemäß Anspruch 1 oder 2, wobei der röhrenförmige Körper (101) ein distales und ein proximales axiales Ende umfasst, wobei das proximale Ende mit einem speiseröhrenseitigen Halteflansch (102) versehen ist und das distale Ende mit einem luftröhrenseitigen Halteflansch (103) versehen ist, und wobei zumindest ein Abschnitt des röhrenförmigen Körpers (101), des speiseröhrenseitigen Halteflansches (102) und/oder des luftröhrenseitigen Halteflansches (103) Silikon umfasst und mindestens ein Abschnitt des Silikons mit Rhamnolipiden beschichtet ist.

12. Stimmprothese (100) gemäß Anspruch 11, wobei der röhrenförmige Körper (101), der speiseröhrenseitige Halteflansch (102) und/oder der luftröhrenseitige Halteflansch (103) aus Silikon hergestellt ist und das Silikon mit Rhamnolipiden beschichtet ist.

## Revendications

1. Dispositif médical comportant des surfaces en silicone irrégulières et/ou incurvées, au moins une partie des surfaces en silicone irrégulières et/ou incurvées étant revêtue de rhamnolipides, le dispositif médical étant une prothèse vocale (100) destinée à être montée dans une fistule entre la trachée et l'œsophage, comprenant un corps tubulaire (101) comportant une lumière, et une valve de prothèse vocale comprenant un disque de valve (105) et un siège de valve (106) comprenant des surfaces d'étanchéité (108), ledit disque de valve (105) et ledit siège de valve (106) étant disposés dans la lumière du corps tubulaire (101) et contrôlant la communication à travers ladite lumière par interaction entre ledit disque de valve (105) et ledit siège de valve (106), au moins une des surfaces d'étanchéité (108) de la valve de prothèse vocale et/ou au moins une des brides de retenue (102, 103) étant des surfaces en silicone revêtues de rhamnolipides, les rhamnolipides étant liés de façon covalente aux surfaces par le biais d'un groupe de liaison comprenant une liaison amide, le groupe de liaison étant -O-Si-(CH2)3-N-CO- ou -O-(CH2)3-N-CO-, et les rhamnolipides comprenant des mono-rhamnolipides pour 70-85 % (poids/poids) et des di-rhamnolipides pour 15-30 % (poids/poids), la quantité totale de mono-rhamnolipides et de di-rhamnolipides représentant 100 % (poids/poids), et les rhamnolipides présentant une pureté d'au moins de 85 %.

2. Dispositif médical selon la revendication 1, dans lequel la densité surfacique de rhamnolipides est supérieure à 4,5×10¹⁵ rhamnolipides par cm², par exemple supérieure à 5×10¹⁵ rhamnolipides par cm², par exemple supérieure à 6×10¹⁵ rhamnolipides par cm², par exemple supérieure à 7×10¹⁵ rhamnolipides par cm², par exemple supérieure à 8×10¹⁵ rhamnolipides par cm².

3. Procédé de revêtement d'un dispositif médical comportant des surfaces en silicone irrégulières et/ou incurvées, le dispositif médical étant la prothèse vocale de la revendication 1 ou 2 avec des rhamnolipides, le procédé comprenant les étapes suivantes :
1) activation des surfaces en silicone qui doivent être revêtues par introduction de groupes amino libres ; et
2) liaison covalente de rhamnolipides auxdites surfaces en silicone.

4. Procédé selon la revendication 3, dans lequel l'étape 1) comprend :
- la soumission de la surface qui doit être revêtue à des monomères de (3-aminopropyl)triéthoxysilane (APTES) ou des monomères de cyclopropylamine.

5. Procédé selon la revendication 3 ou 4, dans lequel l'étape 1) comprend les étapes suivantes :
- soumission de la surface en silicone qui doit être revêtue à une décharge plasma à la pression atmosphérique ou sous vide ;
et
- soumission de la surface en silicone qui doit être revêtue à une solution de monomères de (3-aminopropyl)triéthoxysilane (APTES) ou de monomères de cyclopropylamine ;
et éventuellement :
- soumission de la surface en silicone qui doit être revêtue à une température de 50 à 150 °C, par exemple 70 à 130 °C, par exemple 80 à 120 °C, de préférence 90 à 110 °C, par exemple 95 à 105 °C, mieux encore environ 100 °C, sur une durée de 5 à 45 minutes, de préférence 10 à 30 minutes, par exemple 15 à 25 minutes, mieux encore environ 20 minutes.

6. Procédé selon la revendication 3 ou 4, dans lequel l'étape 1) comprend les étapes suivantes :
- soumission de la surface en silicone qui doit être revêtue à une décharge plasma à la pression atmosphérique ou sous vide d'argon combiné à des monomères de (3-aminopropyl)triéthoxysilane (APTES) ou d'argon combiné à des monomères de cyclopropylamine ;
et éventuellement :
- soumission de la surface en silicone qui doit être revêtue à une température de 90 à 110 °C, par exemple 95 à 105 °C, mieux encore environ 100 °C, sur une durée de 5 à 45 minutes, de préférence 10 à 30 minutes, par exemple 15 à 25 minutes, mieux encore environ 20 minutes.

7. Procédé selon l'une quelconque des revendications 3 à 6 dans lequel, après l'étape 1), la densité surfacique de groupes amino est supérieure à 6×10¹⁵ groupes amino par cm², par exemple supérieure à 7×10¹⁵ groupes amino par cm², par exemple supérieure à 8×10¹⁵ groupes amino par cm².

8. Procédé selon l'une quelconque des revendications 3 à 7, dans lequel l'étape 2) est réalisée au moyen de la chimie des carbodiimides.

9. Procédé selon l'une quelconque des revendications 3 à 8, dans lequel l'étape 2 comprend les étapes suivantes :
2a) mélange d'une solution aqueuse de rhamnolipides avec une solution aqueuse comprenant du N-éthyl-N'-(3-diméthylaminopropyl)carbodiimide et du N-hydroxysuccinimide à pH 4,5 à 6, de préférence à pH 5,5 ;
2b) augmentation du pH jusqu'à 7,4 par ajout de NaOH ; et
2c) soumission de la surface en silicone qui doit être revêtue au mélange obtenu à l'étape 2b) pendant 1 à 24 h, de préférence pendant 8 à 16 h, mieux encore 10 à 14 h, par exemple environ 12 h, à une température de 4 à 25 °C.

10. Procédé selon l'une quelconque des revendications 3 à 9, comprenant en outre l'étape suivante :
3) rinçage de la surface en silicone revêtue obtenue à l'issue de l'étape 2) avec de l'eau distillée ;
et
4) séchage de la surface en silicone revêtue sous vide à température ambiante.

11. Prothèse vocale (100) selon la revendication 1 ou 2, dans laquelle le corps tubulaire (101) comprend une extrémité axiale distale et une extrémité axiale proximale, dans laquelle l'extrémité proximale est pourvue d'une bride de retenue œsophagienne (102) et l'extrémité distale est pourvue d'une bride de retenue trachéale (103), et dans laquelle au moins une partie du corps tubulaire (101), de la bride de retenue œsophagienne (102) et/ou de la bride de retenue trachéale (103) comprend du silicone et au moins une partie dudit silicone est revêtue de rhamnolipides.

12. Prothèse vocale (100) selon la revendication 11, dans laquelle le corps tubulaire (101), la bride de retenue œsophagienne (102) et/ou la bride de retenue trachéale (103) sont constitués de silicone et ledit silicone est revêtu de rhamnolipides.
